(19) Europäisches Patentamt
European Patent Office
Office européen des brevets

(11) **EP 2 531 522 B1**

(12) **FASCICULE DE BREVET EUROPEEN**

(45) Date de publication et mention de la délivrance du brevet:
**23.07.2014 Bulletin 2014/30**

(21) Numéro de dépôt: **11706354.5**

(22) Date de dépôt: **03.02.2011**

(51) Int Cl.:
*C07K 14/435* *(2006.01)*    *A61P 31/04* *(2006.01)*
*A61P 31/12* *(2006.01)*    *A61K 38/00* *(2006.01)*
*A61P 33/02* *(2006.01)*

(86) Numéro de dépôt international:
**PCT/IB2011/050465**

(87) Numéro de publication internationale:
**WO 2011/095939 (11.08.2011 Gazette 2011/32)**

(54) **PEPTIDES ANTIMICROBIENS DE LA FAMILLE DES CECROPINES ET LEURS UTILISATIONS THERAPEUTIQUE**

ANTIMIKROBIELLE PEPTIDE DER CECROPIN-FAMILIE UND IHRE THERAPEUTISCHE VERWENDUNG

ANTIMICROBIAL PEPTIDES OF THE CECROPIN FAMILY AND THERAPEUTIC USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.02.2010 FR 1000434**

(43) Date de publication de la demande:
**12.12.2012 Bulletin 2012/50**

(73) Titulaire: **Institut de Recherche pour le Développement ( IRD)**
**13572 Marseille Cédex 02 (FR)**

(72) Inventeurs:
• **MISSE BRUMAS, Marthe Dorothée**
**F-34820 Teyran (FR)**
• **LUPLERTLOP, Natthanej**
**Thaïlande 11000 (TH)**
• **YSSEL, Hans**
**F-34160 Sussargues (FR)**
• **THOMAS, Frédéric**
**F-34090 Montpellier (FR)**
• **RENAUD, François**
**F-34090 Montpellier (FR)**

(74) Mandataire: **Peaucelle, Chantal et al**
**Cabinet Armengaud Aîné**
**3, Avenue Bugeaud**
**75116 Paris (FR)**

(56) Documents cités:
**WO-A1-03/008442**

• **DATABASE UniProt [Online] 25 juillet 2006 (2006-07-25), Nene V. et al.: "Genome sequence of Aedes aegypti", XP002598498, extrait de EBI Database accession no. Q17NR1**
• **LOWENBERGER C ET AL: "Antimicrobial Activity Spectrum, cDNA Cloning, and mRNA Expression of a Newly Isolated Member of the Cecropin Family from Mosquito Vector Aedes aegypti", JOURNAL OF BIOLOGICAL CHEMISTRY, AMERICAN SOCIETY FOR BIOCHEMISTRY AND MOLECULAR BIOLOGY, INC, US LNKD- DOI: 10.1074/JBC.274.29.20092, vol. 274, no. 29, 16 juillet 1999 (1999-07-16), pages 20092-20097, XP002165176, ISSN: 0021-9258**
• **SUN D. ET AL.: "peptide sequence of an antibiotic cecropin from the vector mosquito, Aedes albopictus", BIOCHEM. BIOPHYS. RES. COMM., vol. 249, 1998, pages 410-415, XP002598499,**

**EP 2 531 522 B1**

SEQUENCE LISTING

<110> INSTITUT DE RECHERCHE POUR LE DEVELOPPEMENT (IRD)

<120> Peptides antimicrobiens et leurs utilisations en thérapeutique

<130> CP 63083-3648

<150> FR 10/00434
<151> 2010-02-03

<160> 3

<170> PatentIn version 3.5

<210> 1
<211> 61
<212> PRT
<213> Artificial sequences

<220>
<223> Antimicrobial peptides

<400> 1

```
        Met Asn Met Asn Phe Thr Lys Leu Phe Ala Ile Val Leu Leu Ala Ala
        1               5               10              15


        Leu Val Leu Leu Gly Gln Thr Glu Ala Gly Gly Leu Lys Lys Leu Gly
                    20              25              30


        Lys Lys Leu Glu Gly Ala Gly Lys Arg Val Phe Lys Ala Ser Glu Lys
                35              40              45


        Ala Leu Pro Val Val Val Gly Ile Lys Ala Ile Gly Lys
            50              55              60
```

<210> 2
<211> 36
<212> PRT
<213> Artificial sequences

<220>
<223> antimicrobial peptides

<400> 2

```
        Gly Gly Leu Lys Lys Leu Gly Lys Lys Leu Glu Gly Ala Gly Lys Arg
        1               5               10              15


        Val Phe Lys Ala Ser Glu Lys Ala Leu Pro Val Val Val Gly Ile Lys
                    20              25              30


        Ala Ile Gly Lys
                35
```

<210> 3
<211> 25
<212> PRT
<213> Artificial sequences

<220>
<223> antimicrobial peptides

<400> 3

```
        Met Asn Met Asn Phe Thr Lys Leu Phe Ala Ile Val Leu Leu Ala Ala
        1               5               10              15


        Leu Val Leu Leu Gly Gln Thr Glu Ala
                    20              25
```

**Description**

La présente invention concerne des peptides antimicrobiens pour leur utilisation comme médicament à des fins de lutte antibactérienne, antivirale et/ou antiparasitaire.

Les peptides antimicrobiens désignent un vaste ensemble de peptides (plus de 500 peptides ont été identifiés à ce jour) comportant en moyenne de 20 à 50 acides aminés, qui partagent un caractère cationique et des propriétés amphiphiles. Leur diversité structurale est grande, à l'origine de trois grandes familles de peptides antimicrobiens cationiques. Les cécropines furent les premiers peptides microbiens cationiques entièrement caractérisés, à partir des nymphes du papillon Hyalophora cecropia, au début des années 80 par H. Boman (J. Intern. Med. 2003 ; 254 :197-215).

Les peptides antimicrobiens cationiques sont considérés comme l'un des éléments clés du système immunitaire inné, système qui assure la première ligne de défense des organismes multicellulaires.

L'utilisation de peptides antimicrobiens d'insecte, qui sont des substances naturelles produites par les insectes, ainsi que l'utilisation de leurs dérivés sont actuellement en cours de développement clinique, essentiellement sous forme topique (diminution de la flore microbienne locale afin de prévenir la survenue des infections : « Andrès et Dimarcq, Med Mal Infect 2007 ; 37 :194-199 »).

Tant en santé humaine qu'animale, face à l'émergence de nouveaux pathogènes et surtout de microorganismes multirésistants, le besoin de découvrir des molécules qui permettent de lutter contre les infections aussi bien bactériennes, virales que parasitaires, est grandissant, et les industriels de la pharmacie s'intéressent de plus en plus à des molécules qui ont un large spectre d'activité.

Ainsi, la présente invention a notamment pour but de fournir des molécules qui présentent un large spectre d'activité.

Plus particulièrement, un des buts de l'invention est de fournir des molécules qui permettent de lutter à la fois contre les infections bactériennes, virales et parasitaires, ou au moins contre deux desdites infections.

Il a été découvert de manière surprenante par les Inventeurs, que des peptides antimicrobiens naturels d'insectes, à savoir des peptides antimicrobiens d'*Aedes Aegypti* permettaient de remplir le but recherché par la présente invention.

Parmi les peptides antimicrobiens d'*Aedes Aegypti* objets de la présente invention, le peptide de séquence SEQ ID N°1 tel que défini ci-dessous a déjà été décrit dans la littérature comme appartenant à la famille des cécropines et comme étant un peptide antibactérien putatif (voir base de données « *aaegypti. vectorbase.org*» et « *UniProtKB/TrEMBL* »). Cependant aucune fonction n'a été démontrée à ce jour concernant ce peptide.

Concernant les peptides de la famille des cécropines, certains d'entres eux ont été décrits dans la littérature comme présentant une activité pour le traitement de la leishmaniose. Ainsi, les documents « WO03008442 », « Biochem J. 1998 Feb 15, 330 (Pt 1): 453-60 », « Biochem J. 2003 Oct 1, 375 (Pt 1) : 221-30 », « Protein Pept Lett. 2004 Apr, 11(2) : 115-24 », « Antimicrob Agents Chemother. 2004 Feb, 48 (2) : 641-3 » et « Antimicrob Agents Chemother. 2001 Sep, 45(9) : 2441-9 » décrivent tous l'utilisation de peptides hybrides cécropine-mellitine pour le traitement de la leishmaniose.

Le peptide de séquence SEQ ID N°1 est le peptide immature qui comporte le peptide signal. Le clivage du peptide signal permet d'obtenir le peptide sécrété ou peptide mature.

Les Inventeurs ont pu déterminer que le peptide signal correspondait au peptide de séquence SEQ ID N°3 tel que défini ci-dessous et ils ont ainsi pu déterminer que le peptide mature ou sécrété correspondait au peptide de séquence SEQ ID N°2 tel que défini ci-dessous.

De façon habituelle dans l'état de l'art, les tests d'efficacité des peptides sont réalisés sur les peptides sécrétés, c'est-à-dire sur les peptides dépourvus de leur peptide signal. Ainsi, les tests d'activités effectués dans la présente demande de brevet ont été effectués sur le peptide de séquence SEQ ID N°2.

Cependant, une originalité du travail des Inventeurs a été de tester également l'efficacité d'un peptide antimicrobien naturel d'insecte avec son peptide signal, ce qui n'avait jamais été réalisé auparavant.

Ainsi, il a été découvert de façon surprenante par les Inventeurs que le peptide de séquence SEQ ID N°1 était particulièrement intéressant sur le plan de son activité puisqu'il présente à la fois des activités aussi bien antibactériennes, antiparasitaires et antivirales.

Il a ainsi pu être déterminé que le peptide immature (de séquence SEQ ID N°1) ne se comportait pas de la même façon que le peptide mature (de séquence SEQ ID N°2). En effet, le peptide de séquence SEQ ID N°1 présente une activité virale plus prononcée que celle du peptide de séquence SEQ ID N°2 tandis que le peptide de séquence SEQ ID N°2 présente une activité antibactérienne plus prononcée que celle du peptide de séquence SEQ ID N°1. De plus, le peptide de séquence SEQ ID N°2 présente des activités antibactériennes et antivirales mais ne présente pas d'activité antiparasitaire, contrairement au peptide SEQ ID N°1 qui présente à la fois des activités antibactériennes, antivirales et antiparasitaires.

Le peptide signal de séquence SEQ ID N°3 se révèle donc intéressant quant à sa fonctionnalité puisqu'il constitue la seule différence entre le peptide de séquence SEQ ID N°2 et celui de séquence SEQ ID N°1.

Ainsi la présente invention concerne aussi bien les peptides antimicrobiens d'*Aedes Aegypti* avec ou sans peptide signal, que le peptide signal en lui-même.

La séquence SEQ ID N°1 est la suivante :

MNMNFTKLFAIVLLAALVLLGQTEAGGLKKLGKKLEGAGKRVFKASEKALPVVVGIKAIGK

La séquence SEQ ID NO 2 est :

GGLKKLGKKLEGAGKRVFKASEKALPVVVGIKAIGK

La séquence SEQ ID NO°3 est :

MNMNFTKLFAIVLLAALVLLGQTEA

La présente invention a plus particulièrement pour objet un peptide antimicrobien caractérisé en ce qu'il présente la séquence SEQ ID N°1 ou la séquence SEQ ID N°2, la séquence SEQ ID N°2 représentant un fragment de la séquence SEQ ID N°1, pour utilisation comme médicament.

Selon l'invention, ledit médicament comprend une quantité thérapeutiquement efficace d'au moins un peptide tel que défini ci-dessus, en association avec un véhicule pharmaceutiquement acceptable.

De façon avantageuse selon l'invention, le médicament est préparé pour une administration par voie injectable ou pour une administration sous forme topique.

Plus particulièrement, le peptide tel que défini ci-dessus, de séquence SEQ ID N°1 ou SEQ ID N°2, est utilisé pour le traitement d'infections bactériennes et/ou virales.

Selon un mode de réalisation avantageux de l'invention, le peptide tel que défini ci-dessus, de séquence SEQ ID N°1 ou SEQ ID N°2, est plus particulièrement utilisé pour le traitement d'infections bactériennes, et notamment celles dues à des bactéries à *Gram positif* et/ou à *Gram négatif.*

Selon un autre mode de réalisation avantageux de l'invention, le peptide tel que défini ci-dessus, de séquence SEQ ID N°1 ou SEQ ID N°2, est plus particulièrement utilisé pour le traitement d'infections virales, et notamment celles dues au virus de la Dengue et/ou au virus du Chikungunya.

De manière avantageuse selon l'invention, le peptide tel que défini ci-dessus, de séquence SEQ ID N°1 ou SEQ ID N°2, est utilisé pour le traitement d'infections bactériennes et virales.

Selon un autre mode de réalisation avantageux, l'invention concerne plus particulièrement le peptide antimicrobien de séquence SEQ ID N°1, pour le traitement d'infections parasitaires, et notamment celles dues au parasite *Leishmania braziliensis* et/ou au parasite *Leishmania infantum.*

De manière avantageuse selon l'invention, le peptide de séquence SEQ ID N°1 est utilisé pour le traitement d'infections bactériennes, virales et parasitaires.

La présente invention concerne encore une composition pharmaceutique caractérisée en ce qu'elle comporte une quantité thérapeutiquement efficace d'un peptide antimicrobien présentant la séquence SEQ ID N°1 ou SEQ ID N°2 telles que définies ci-dessus, en association avec un véhicule pharmaceutiquement acceptable.

De manière avantageuse selon l'invention, la composition pharmaceutique est destinée à une administration par voie injectable ou sous forme topique.

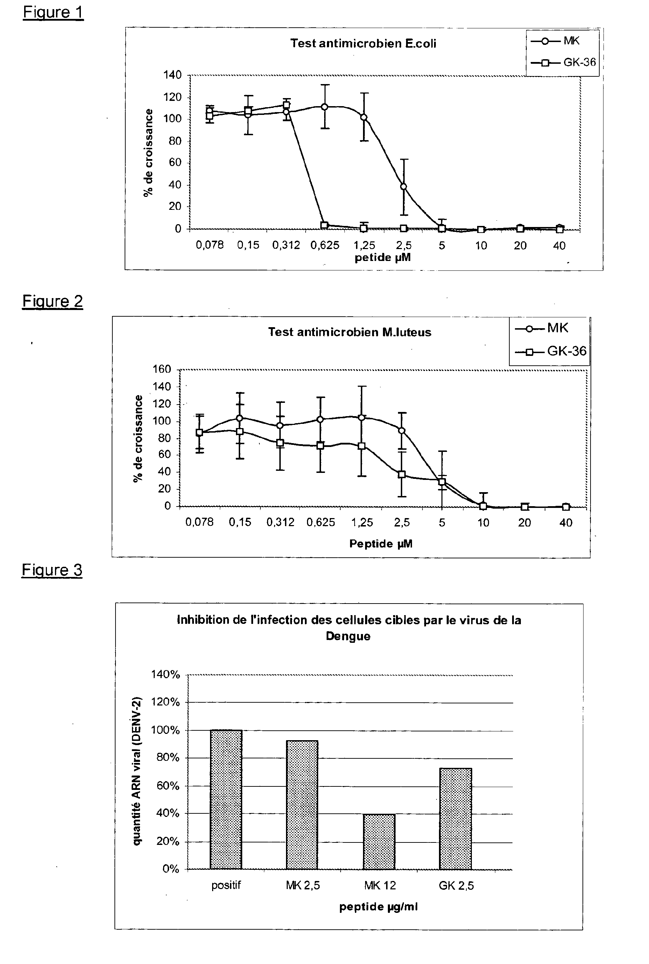

D'autres caractéristiques et avantages de l'invention sont donnés dans la description qui suit avec référence aux figures 1 à 7.

La figure 1 représente le test antimicrobien avec la bactérie Gram négatif *E. coli* et les peptides « MK61 » (peptide de séquence SEQ ID N°1) et « GK36 » (peptide de séquence SEQ ID N°2). La figure 2 représente le test antimicrobien avec la bactérie Gram positif *Micrococcus Luteus* et les peptides MK61 et GK36.

L'axe des ordonnées de chacune des figures 1 et 2 représente le pourcentage de croissance bactérienne et l'axe des abscisses la concentration en peptides ($\mu$M). Le symbole -o- représente le peptide MK61 et le symbole -□- le peptide GK36.

La figure 3 est un histogramme qui représente l'inhibition de l'infection des cellules dendritiques par le virus de la dengue sérotype 2 « DENV-2 » à l'aide des peptides MK61 et GK36.

L'axe des ordonnées représente le pourcentage d'infection des cellules dendritiques par le virus « DENV-2 » et l'axe des abscisses la concentration en peptides ($\mu$M).

La colonne la plus à gauche représente le témoin positif lorsque 100% des cellules sont infectées par DENV-2. Les deux colonnes suivantes représentent respectivement les résultats obtenus pour le peptide MK61 à une concentration de 2,5 $\mu$g/ml et à une concentration de 12 $\mu$g/ml. La colonne la plus à droite représente le résultat obtenu pour le peptide GK36 à une concentration de 2,5 $\mu$g/ml.

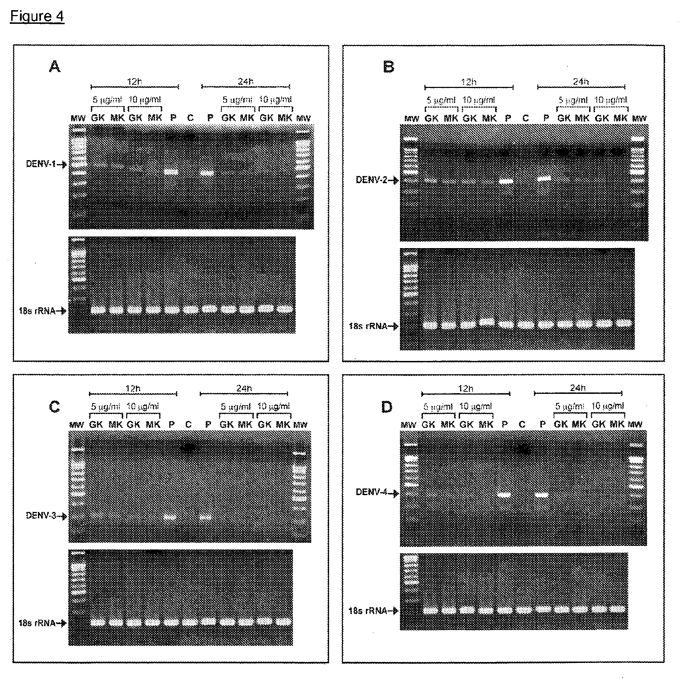

Les figures 4A, 4B, 4C et 4D représentent l'inhibition de l'infection des cellules C6/36 de moustiques *d'Aedes*

*albopictus* par le virus de la dengue (respectivement les virus de sérotype 1, 2, 3 et 4 : « DENV-1 » « DENV-2 », « DENV-3 » et « DENV-4 ») à l'aide des peptides MK61 et GK36. Les peptides MK61 et GK36 sont préparés chacun à des concentrations de 5 $\mu$g/ml et 10 $\mu$g/ml et sont testés au bout de 12 heures et de 24 heures d'incubation des cellules avec le virus.

La figure 5 est un histogramme qui représente l'inhibition de l'infection des cellules HEK-293T par le virus Chikungunya 147-2 à l'aide des peptides MK61 et GK36.

L'axe des ordonnées représente le pourcentage d'infection des cellules HEK-293T par le virus Chikungunya 147-2 et l'axe des abscisses la concentration en peptides ($\mu$M).

Les peptides GK36 et MK61 sont préparés chacun à des concentrations de 10 $\mu$g/ml, 50 $\mu$g/ml et 80 $\mu$g/ml.

Les figures 6 et 7 représentent le test antiparasitaire avec le peptide MK61 et respectivement le parasite *Leishmania braziliensis* et *Leishmania infantum.*

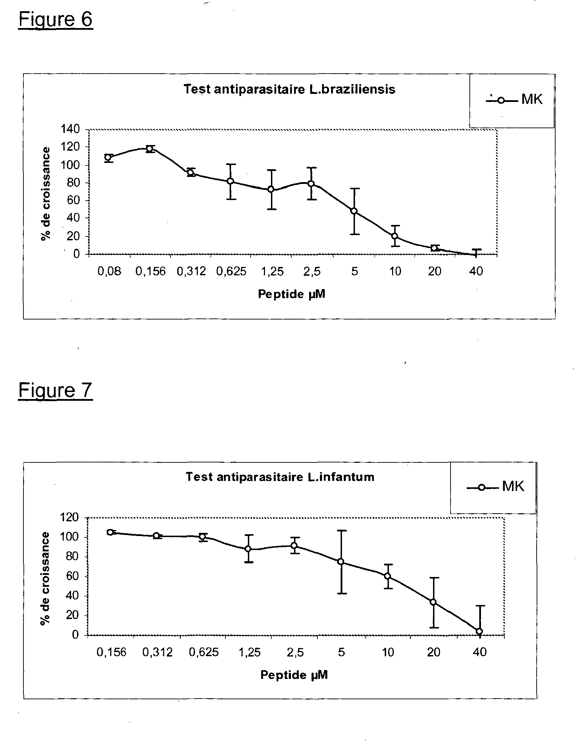

L'axe des ordonnées de chacune des figures 6 et 7 représente le pourcentage de croissance parasitaire et l'axe des abscisses la concentration en peptides ($\mu$M). Le symbole -o- représente le peptide MK61.

L'exemple ci-après illustre l'invention, il ne la limite en aucune façon.

## EXEMPLE 1 : TESTS ANTIBACTERIENS, ANTIVIRAUX ET ANTIPARASITAIRES DES PEPTIDES DE SEQUENCE « SEQ ID N°1 » ET « SEQ ID N°2 ».

### A. SYNTHESE DES PEPTIDES

Les peptides de séquence SEQ ID N°1 (dénommé « MK61 » ou « MK » dans ce qui suit) et de séquence SEQ ID N°2 (dénommé « GK36 » ou « GK » dans ce qui suit) ont été préparés par la société Proteogenix SA (France).

Ils sont synthétisés par voie chimique, purifiés par chromatographie liquide haute performance (HPLC) et contrôlés par spectrométrie de masse. Le degré de pureté de chaque peptide est supérieur à 95% et les poids moléculaires des peptides GK36 et MK61 sont respectivement de 3676,57 g/mol et de 6380,93 g/mol.

### B. TESTS ANTIBACTERIENS

Les bactéries *Escherichia coli* (Gram négatif) et *Micrococcus luteus* (Gram positif) sont cultivées dans un milieu de culture LB (Luria-Bertani) avec et sans les peptides GK36 et MK61 à tester. La croissance bactérienne est contrôlée après une nuit d'incubation à 37°C en mesurant le changement de la valeur de l'absorbance à 600 nanomètres (A600) à l'aide d'un spectrophotomètre pour microplaques. Les peptides MK61 et GK36 sont incubés pendant 24 heures avec les bactéries Gram négatif et Gram positif. La concentration minimale inhibitrice (CMI) des peptides synthétiques GK36 et MK61 représente la concentration la plus faible de peptides qui est suffisante pour inhiber complètement la croissance bactérienne.

Les résultats obtenus sont illustrés à la figure 1 pour la bactérie *Escherichia coli* (Gram négatif) et à la figure 2 pour la bactérie *Micrococcus luteus* (Gram positif).

Conclusion :

Les peptides GK36 et MK61 ont une activité marquée contre la bactérie Gram négatif *E. coli* avec des CMI respectivement de 0,6 $\mu$M est 5 $\mu$M (cf. figure 1).

Les peptides GK36 et MK61 ont également une bonne activité contre la bactérie Gram positif *Micrococcus Luteus* avec des CMI de 10 $\mu$M (cf. figure 2).

Les peptides GK36 et MK61 présentent donc une activité antibactérienne satisfaisante.

### C. TESTS ANTIVIRAUX

1) VIRUS DE LA DENGUE (dengue sérotype 2)

(a) Inhibition de l'infection des cellules dendritiques par le virus de la dengue

Les cellules dendritiques sont les principales cibles du virus de la dengue (« Navarro-Sanchez et al., EMBO Rep. 2003 Jul., 4 (7) : 723-8 » et « Tassaneetrithep et al., J Exp Med., 2003, Apr. 7, 197(7): 823-9 »).

Les cellules dendritiques sont incubées pendant 3 heures à 37°C en présence du virus de la dengue (elles sont infectées à un « MOI » (« Multiplicity of infection ») de 0,4) avec et sans les peptides MK61 et GK36 à tester qui sont préparés à des concentrations de 2,5 $\mu$g/ml et 12 $\mu$g/ml pour MK61 et 2,5 $\mu$g/ml pour GK36. Le surnageant est ensuite retiré et les cellules sont mises en présence ou non de peptide pendant 48 h.

Des analyses de PCR en temps réel sont ensuite effectuées. La viabilité cellulaire est évaluée par cytométrie en flux et aucune toxicité cellulaire n'est observée.

Les résultats obtenus sont illustrés à la figure 3.

Conclusion :

Avec le peptide MK61 à 12 $\mu$g/ml on passe de 100% d'infection à 40% d'infection. Ainsi le peptide MK61 à 12 $\mu$g/ml entraîne une inhibition de 60% de l'infection des cellules dendritiques. On note une inhibition dose dépendante pour le peptide MK61.

Le peptide GK36 à 2,5 $\mu$g/ml entraîne une inhibition de 35% de l'infection.

Les peptides GK36 et MK61 présentent donc une activité antivirale satisfaisante.

(b) Inhibition de l'infection des cellules C6/36 par le virus de la dengue

Les cellules C6/36 de moustiques *d'Aedes albopictus* sont incubées pendant 1 heure à 4°C avec les différents virus de la dengue ((sérotype 1) « DENV-1 » ou (sérotype 2) « DENV-2 » ou (sérotype 3) « DENV-3 » ou (sérotype 4) « DENV-4 ») (avec des « PFU » (« Plaque Forming Units » ou « Unités formant des phages de lyse ») de 1). Les cellules sont ensuite lavées trois fois et incubées pendant 12 heures ou 24 h à 28°C en présence ou non des peptides GK36 et MK61 à tester qui sont préparés chacun à des concentrations de 5 $\mu$g/ml ou de 10 $\mu$g/ml.

Des analyses de RT-PCR sont ensuite effectuées sur les culots cellulaires afin de détecter la présence du virus de la dengue.

Les résultats obtenus sont représentés à la figure 4 (A, B, C ou D).

La lettre P signifie que le contrôle est positif, c'est-à-dire que les cellules sont infectées.

La lettre C signifie que le contrôle est négatif, c'est-à-dire que les cellules récupérées au bout de 24 heures ne sont pas infectées.

La lettre M désigne le marqueur de poids moléculaire.

Les résultats montrent une forte réduction de la production de virus en présence des peptides GK36 et MK61 et ceci de manière dose dépendante (on observe une meilleure inhibition de l'infection à 10 $\mu$g/ml qu'à 5 $\mu$g/ml). On constate également qu'on a un meilleur effet inhibiteur des peptides à 24 h d'exposition par rapport au temps de 12 h. Au vu de l'intensité des bandes, on constate que le peptide MK est légèrement plus efficace que le peptide GK.

La visibilité par RT-PCR du gène 18SrRNA (gène de ménage) sur les culots cellulaires montre que toutes les conditions présentent la même quantité d'ARN.

Conclusion :

Les peptides GK36 et MK61 inhibent l'infection des cellules C6/36 de moustiques *d'Aedes albopictus* par le virus de la dengue (virus dengue-1, virus dengue-2, virus dengue-3 et virus dengue-4).

Cet effet est dose dépendant et est plus prononcé au bout de 24 h. On note également que le peptide MK61 est encore plus efficace que le peptide GK36.

2) VIRUS DU CHIKUNGUNYA

Les cellules, qui proviennent de la lignée cellulaire humaine HEK-293T (Human Embryonic Kidney 293), sont incubées pendant 2 heures à 37°C en présence de virus CHIKV-147-2 GFP (elles sont infectées à une « MOI » de 1) avec et sans les peptides GK36 et MK61 à tester qui sont préparés chacun à des concentrations de 10 $\mu$g/ml, 50 $\mu$g/ml et 80 $\mu$g/ml. Le surnageant est ensuite retiré et les cellules sont mises en présence ou non de peptides pendant 48 heures. Le pourcentage de cellules infectées correspond au pourcentage de cellules GFP (« Green Fluorescent Protein ») visualisé en cytométrie en flux (10000 cellules ont été comptées par condition).

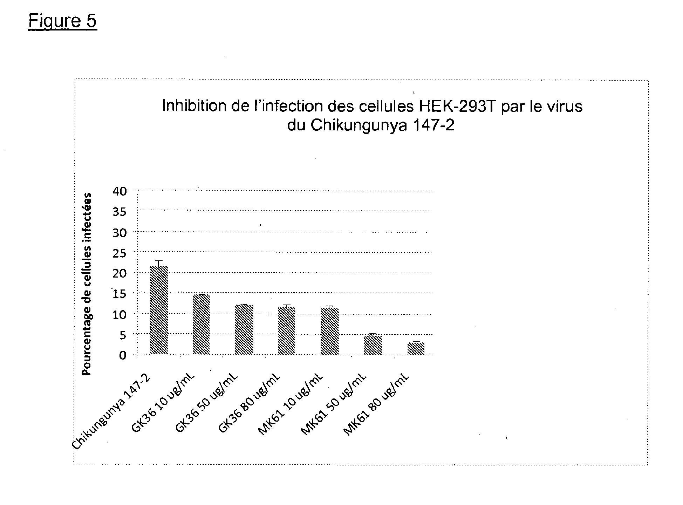

Les résultats obtenus sont représentés à la figure 5.

Conclusion :

Le peptide MK61 inhibe très efficacement l'infection des cellules HEK-293T et de manière dose dépendante, comme on a pu l'observer pour le virus de la dengue.

Le peptide GK36 inhibe également l'infection des cellules HEK-293T et de manière dose dépendante.

Les résultats obtenus aussi bien sur le virus du chikungunya que sur le virus de la dengue montrent donc que les peptides de séquence SEQ ID N°1 et SEQ ID N°2 sont des inhibiteurs puissants de l'infection virale.

## D. TESTS ANTIPARASITAIRES

Détermination de l'activité leishmaniose

Des promastigotes de *Leishmania infantum* (souche MHOM/MA/67/ITMAP-263) et de *Leishmania braziliensis* (souche MHOM/BR/75M2904) exprimant le gène luciférase ont été utilisés pour tester les activités anti-leishmanioses du peptide antimicrobien MK61 de la manière précédemment décrite (Sereno et al., Antimicrob Agents Chemother. 2001 Apr;45(4):1168-73). En bref, des formes promastigotes ont été inoculées à raison de $10^5$ dans 100 µl de milieu, dans des plaques de 96 puits, puis le peptide a été ajouté 4 h après et les plaques ont été à nouveau incubées pendant 72 h.

L'activité luciférase a ensuite été évaluée afin de déterminer le pourcentage de croissance des parasites. Les résultats sont exprimés en indice « RLU » « Relative Light Unit ».

Indice RLU = (RLU dans les puits traités / RLU dans les puits contrôlés) x 100.

Les résultats obtenus sont illustrés dans les figures 6 et 7 qui représentent l'incubation des promastigotes exprimant le gène de la luciférase pendant 72h à 37°C avec le peptide MK61.

Conclusion :

Le peptide MK61 présente la capacité de tuer les leishmanies. Plus particulièrement ce peptide présente une activité antiparasitaire sur les deux souches de leishmanies (*Leishmania infantum* et *Leishmania braziliensis*) avec des concentrations minimales inhibitrices de 20 à 40 µM.

**Revendications**

1. Peptide antimicrobien **caractérisé en ce qu'**il présente la séquence SEQ ID N°1 ou la séquence SEQ ID N°2, la séquence SEQ ID N° 2 représentant un fragment de la séquence SEQ ID N°1, pour utilisation comme médicament.

**2.** Peptide tel que défini dans la revendication 1, pour utilisation comme médicament pour le traitement d'infections bactériennes et/ou virales.

**3.** Peptide pour utilisation selon la revendication 2, pour le traitement d'infections bactériennes.

**4.** Peptide pour utilisation selon la revendication 3, pour le traitement d'infections dues à des bactéries à *Gram positif* et/ou à *Gram négatif.*

**5.** Peptide pour utilisation selon la revendication 2, pour le traitement d'infections virales.

**6.** Peptide pour utilisation selon la revendication 5, pour le traitement d'infections dues au virus de la Dengue et/ou au virus du Chikungunya.

**7.** Peptide pour utilisation selon l'une quelconque des revendications 2 à 6, pour utilisation comme médicament pour le traitement d'infections bactériennes et virales.

**8.** Peptide antimicrobien pour utilisation pour le traitement d'infections parasitaires, **caractérisé en ce qu'**il présente la séquence SEQ ID N°1.

**9.** Peptide pour utilisation selon la revendication 8, pour le traitement d'infections dues au parasite *Leishmania braziliensis* et/ou au parasite *Leishmania infantum.*

**10.** Peptide pour utilisation selon les revendications 8 ou 9, pour le traitement d'infections parasitaires.

**11.** Composition pharmaceutique **caractérisée en ce qu'**elle comporte une quantité thérapeutiquement efficace d'un peptide selon l'une quelconque des revendications 1 à 10, en association avec un véhicule pharmaceutiquement acceptable.

**12.** Composition selon la revendication 11, pour une administration par voie injectable.

**13.** Composition selon la revendication 11, pour une administration sous forme topique.


**Patentansprüche**

**1.** Antimikrobielles Peptid, **dadurch gekennzeichnet, dass** es die Sequenz SEQ ID N°1 oder die Sequenz SEQ ID N°2 aufweist, wobei die Sequenz SEQ ID N°2 ein Fragment der Sequenz SEQ ID N°1 darstellt, zur Verwendung als Medikament.

**2.** Peptid wie in Anspruch 1 definiert, zur Verwendung als Medikament für die Behandlung von bakteriellen und/oder viralen Infektionen.

**3.** Peptid zur Verwendung nach Anspruch 2, für die Behandlung von bakteriellen Infektionen.

**4.** Peptid zur Verwendung nach Anspruch 3, für die Behandlung von Infektionen, die durch grampositive und/oder gramnegative Bakterien bedingt sind.

**5.** Peptid zur Verwendung nach Anspruch 2, für die Behandlung von viralen Infektionen.

**6.** Peptid zur Verwendung nach Anspruch 5, für die Behandlung von Infektionen, die durch das Dengue-Virus und/oder das Chikungunya-Virus bedingt sind.

**7.** Peptid zur Verwendung nach einem der Ansprüche 2 bis 6, zur Verwendung als Medikament für die Behandlung von bakteriellen und viralen Infektionen.

**8.** Antimikrobielles Peptid zur Verwendung für die Behandlung von parasitären Infektionen, **dadurch gekennzeichnet, dass** es die Sequenz SEQ ID N°1 aufweist.

**9.** Peptid zur Verwendung nach Anspruch 8, für die Behandlung von Infektionen, die durch den Parasit *Leishmania braziliensis* und/oder den Parasit *Leishmania infantum* bedingt sind.

**10.** Peptid zur Verwendung nach den Ansprüchen 8 oder 9, für die Behandlung von parasitären Infektionen.

**11.** Pharmazeutische Zusammensetzung, **dadurch gekennzeichnet, dass** sie eine therapeutisch wirksame Menge eines Peptids nach einem der Ansprüche 1 bis 10 in Kombination mit einem pharmazeutisch akzeptablen Trägerstoff umfasst.

**12.** Zusammensetzung nach Anspruch 11, für eine Verabreichung auf dem Injektionsweg.

**13.** Zusammensetzung nach Anspruch 11, für eine Verabreichung in topischer Form.

**Claims**

**1.** Antimicrobial peptide, **characterised in that** it has the sequence SEQ ID No.1 or the sequence SEQ ID No. 2, the sequence SEQ ID No. 2 representing a fragment of the sequence SEQ ID No. 1, for use as a medicament.

**2.** Peptide such as defined in claim 1, for use as drug for the treatment of bacterial and/or viral infections.

**3.** Peptide for use according to claim 2, for the treatment of bacterial infections.

**4.** Peptide for use according to claim 3, for the treatment of infections caused by *Gram positive* and/or *Gram negative* bacteria.

**5.** Peptide for use according to claim 2 for the treatment of viral infections.

**6.** Peptide for use according to claim 5, for the treatment of infections caused by the dengue virus and/or the chikungunya virus.

**7.** Peptide for use according to any one of claims 2 to 6 for use as drug for the treatment of bacterial and viral infections.

**8.** Antimicrobial peptide for use for the treatment of parasitic infections, **characterised in that** it has the sequence SEQ ID No. 1.

**9.** Peptide for use according to claim 8, for the treatment of infections caused by the parasite *Leishmania braziliensis* and/or by the parasite *Leishmania infantum.*

**10.** Peptide according to claim 8 or 9, for the treatment of parasitic infections.

**11.** Pharmaceutical composition, **characterised in that** it comprises a therapeutically effective amount of a peptide according to any one of claims 1 to 10, in association with a pharmaceutically acceptable carrier.

**12.** Composition according to claim 11, for administration by the injectable route.

**13.** Composition according to claim 11, for administration in topical form.

Figure 1

Test antimicrobien E.coli

MK
GK-36

% de croissance

petide µM

Figure 2

Test antimicrobien M.luteus

MK
GK-36

% de croissance

Peptide µM

Figure 3

Inhibition de l'infection des cellules cibles par le virus de la Dengue

quantité ARN viral (DENV-2)

positif    MK 2,5    MK 12    GK 2,5

peptide µg/ml

Figure 4

## Figure 5

Inhibition de l'infection des cellules HEK-293T par le virus du Chikungunya 147-2

## Figure 6

**Test antiparasitaire L.braziliensis**

—o— MK

% de croissance / Peptide µM

## Figure 7

**Test antiparasitaire L.infantum**

—o— MK

% de croissance / Peptide µM

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 03008442 A
- FR 1000434

### Littérature non-brevet citée dans la description

- **H. BOMAN.** *J. Intern. Med.,* 2003, vol. 254, 197-215
- **ANDRÈS ; DIMARCQ.** *Med Mal Infect,* 2007, vol. 37, 194-199
- *Biochem J.,* 15 Février 1998, vol. 330, 453-60
- *Biochem J.,* 01 Octobre 2003, vol. 375, 221-30
- *Protein Pept Lett.,* Avril 2004, vol. 11 (2), 115-24
- *Antimicrob Agents Chemother.,* Février 2004, vol. 48 (2), 641-3
- *Antimicrob Agents Chemother,* 04 Septembre 2001, vol. 5 (9), 2441-9
- **NAVARRO-SANCHEZ et al.** *EMBO Rep,* Juillet 2003, vol. 4 (7), 723-8
- **TASSANEETRITHEP et al.** *J Exp Med.,* 07 Avril 2003, vol. 197 (7), 823-9
- **SERENO et al.** *Antimicrob Agents Chemother.,* Avril 2001, vol. 45 (4), 1168-73